(19) Europäisches Patentamt
European Patent Office
Office européen des brevets

(11) Veröffentlichungsnummer: **0 316 725**
**A2**

(12) # EUROPÄISCHE PATENTANMELDUNG

(21) Anmeldenummer: **88118616.7**

(51) Int. Cl.4: **C07K 1/02 , C07K 5/06 , A61K 37/64**

(22) Anmeldetag: **09.11.88**

(30) Priorität: **17.11.87 DE 3738908**

(43) Veröffentlichungstag der Anmeldung: **24.05.89 Patentblatt 89/21**

(84) Benannte Vertragsstaaten: **BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Keller, Reinhold, Dr.**
**Kaunweg 8**
**D-6232 Bad Soden am Taunus(DE)**
Erfinder: **Müller, Wolfgang, Dr.**
**Theodor-Körner-Strasse 12**
**D-6238 Hofheim am Taunus(DE)**

(54) **Verfahren zur enzymatischen Peptidsynthese mit mindestens einer nicht-proteinogenen Aminsäure sowie die neuen, bei der Synthese anfallenden Peptide.**

(57) Aminosäuren bzw. Peptide lassen sich enzymatisch mit Hilfe von Thermolysin oder Chymotrypsin miteinander verknüpfen, wenn der eine Aminosäurerest proteinogen und der andere Aminosäurerest nicht-proteinogen ist.

EP 0 316 725 A2

Xerox Copy Centre

# Verfahren zur enzymatischen Peptidsynthese mit mindestens einer nicht-proteinogenen Aminosäure sowie die neuen, bei der Synthese anfallenden Peptide

Die vorliegende Erfindung betrifft ein Verfahren zur enzymatischen Verknüpfung von Aminosäuren, wobei mindestens eine Aminosäure nichtproteinogen ist, zu Peptiden, die entweder schon als solche interessante pharmakologische Eigenschaften aufweisen oder aber als Bausteine für längerkettige Peptide eingesetzt werden können, wobei gegebenenfalls durch den gezielten Einsatz von bestimmten Aminosäuren die pharmakologischen Eigenschaften solcher Peptide verändert werden.

Peptide mit interessanten pharmakologischen Eigenschaften, die mit dem erfindungsgemäßen Verfahren synthetisiert werden können, sind beispielsweise Enkephaline (US 4.322.340; oder Helv. Chim. Acta, 64, 2090, 1981), Bradykine (europ. Patentanmeldung EP 128 602) oder Renin-oder ACE-Inhibitoren (z.B. US 4 322 340).

Für die Darstellung solcher Wirkstoffe werden konventionelle chemische Methoden herangezogen. Dies bedingt den Einsatz aufwendiger Schutzgruppentechniken und außerdem besteht bei diesen chemischen Umsetzungen die Gefahr, daß die optisch aktiven Substanzen teilweise racemisiert werden, was meist zu einer Verringerung der pharmakologischen Aktivität führt.

Diese Nachteile entfallen bei dem Einsatz von enzymatischen Methoden für die Peptidsynthese (s. J. of Protein Chem. 4 (1), 1, 1985).
Enzymkatalytische Peptidsynthesen werden bis heute jedoch fast ausschließlich mit proteinogenen Aminosäuren durchgeführt. Eine Ausnahme bildet die Verknüpfung von D-Aminosäuren (J. Org. Chem., 1986, 51, 2728 und Peptides: Structure and function, 1985, 359).

Es wurde nun überraschend gefunden, daß ein nicht proteinogener Aminosäurerest mit einer proteinogen Aminosäure enzymatisch mit Thermolysin oder Chymotrypsin verknüpft werden kann.

Die Erfindung betrifft nun:
Ein Verfahren zur enzymatischen Verknüpfung von Aminosäuren und/ oder Aminosäurederivaten, wovon eine nicht-proteinogen ist, wobei man

a) eine D,L- oder L-Verbindung der Formel I,

$$HB\text{-}R^2N\text{-}\underset{\displaystyle R^1}{HC}\text{-}COOR^3 \qquad (I)$$

in der
$R^1$ ein Rest der Formel

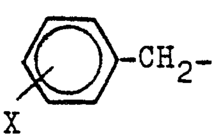

bedeutet, in der
X ein Halogen in ortho- und/oder meta- und/oder para-Position oder ein Nitro- oder Methoxy-Rest in para-Position ist,
oder einen Rest der Formel

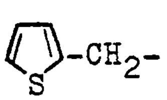, 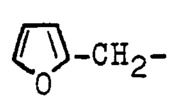, 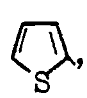, 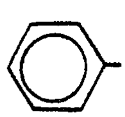, 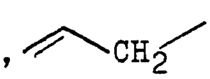 oder

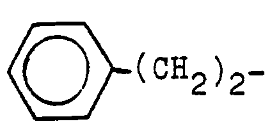 darstellt,

$R^2$ Wasserstoff oder ein Rest der Formel A-CO- ist, worin A Benzyloxy, $C_1$-$C_4$-Alkyl, Phenyl oder $C_1$-$C_4$-Alkoxy bedeutet,
$R^3$ $C_1$-$C_4$-Alkyl oder Benzyl und

$$B\ (HN-\underset{R^4}{CH}-CO)_n \text{ bedeutet,}$$

wobei n 0-10 und $R^4$ eine Peptidkette ist,
mit einer D,L- oder L-Verbindung der Formel II,

$$H_2N - \underset{R^5}{CH} - CO - BR^6 \qquad (II)$$

in der
$R^5$ Wasserstoff, $C_1$-$C_4$-Alkyl oder einen anderen Rest einer proteinogenen Aminosäure bedeutet,
$R^6$ eine Aminogruppe, -NH-($C_1$-$C_4$)-Alkyl oder eine Phenylaminogruppe ist und
B die obengenannte Bedeutung hat,
in Gegenwart von Chymotrypsin zu einer L,L-Verbindung der Formel III,

$$HB - R^2N - \underset{R^1}{HC} - CONH - \underset{R^5}{CH} - CO - BR^6 \qquad (III)$$

in der $R^1$, $R^2$, $R^5$, $R^6$ und B die obengenannte Bedeutung haben, umsetzt
oder

b) eine D,L- oder L-Verbindung der Formel IV,

$$HB - R^8N - \underset{R^7}{CH} - COOH \qquad (IV)$$

in der
$R^7$ ein Rest der Formel HOOC-$(CH_2)_m$- bedeutet, worin m 1 oder 2 ist,
$R^8$ Wasserstoff, $C_1$-$C_4$-Alkyloxycarbonyl oder Benzyloxycarbonyl ist und
B die obengenannte Bedeutung hat
mit einer D,L- oder L-Verbindung der Formel V,

$$H_2N - \underset{R^{10}}{CH} - CO - B - R^9 \qquad (V)$$

in der
$R^{10}$ einen Rest der Formel

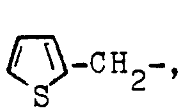,
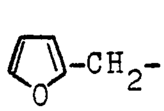
oder
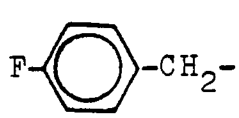

darstellt,
$R^9$ $C_1$-$C_8$-Alkoxy, $NH_2$, NH-($C_1$-$C_4$)-Alkyl oder eine Phenylaminogruppe bedeutet,
in Gegenwart von Thermolysin zu einer L,L-Verbindung der Formel VI,

$$HB - R^8 - N - \underset{R^7}{CH} - CONH - \underset{R^{10}}{CH} - B - R^9 \qquad (VI)$$

in der $R^7$, $R^8$, $R^9$, $R^{10}$ und B die oben angegebene Bedeutung haben, umsetzt.

Weiterhin betrifft die Erfindung die neuen Verbindungen
L-Z-Asp-L-(2-Th-Ala)-$OCH_3$
L-N-Bz-(2-Fu-Ala)-L-Leu-$NH_2$ und
L-Ac-(2-Th-Ala)-L-Leu-$NH_2$.

Ebenso betrifft die Erfindung die Verwendung der erfindungsgemäß hergestellten Peptide zur Herstellung von Bradykinen, Enkephalinen oder Renin- oder ACE-Inhibitoren.

Unter "nicht-proteinogenen" Aminosäuren werden solche Aminosäuren verstanden, die in natürlichen Proteinen nicht vorkommen. Unter "Halogenen" werden Fluor, Chlor, Brom und Jod verstanden, insbesondere Fluor, Chlor und Brom. Alkylreste mit drei und mehr Kohlenstoffatomen können sowohl geradkettig als auch verzweigt sein. Unter einem "proteinogenen" Aminosäurerest ($R^5$) wird ein solcher Rest verstanden, der zusammen mit dem übrigen Teil der Verbindung der Formel II, unter der Annahme, daß das Säureamid ($-CONH_2$) als freie Säure ($-COOH$) vorliegt, eine proteinogene Aminosäure bildet. Also beispielsweise:

$R^5$ = $-CH_3$ (≙ Alanin)
= $-CH(CH_3)-CH_3$ (= Valin)
= $-CH_2-CH(CH_3)-CH_3$ (= Leucin)
= $-CH(CH_3)-CH_2-CH_3$ (= Isoleucin)
= $-CH_2-$ 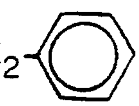 (= Phenylalanin)
= $-CH_2-$ 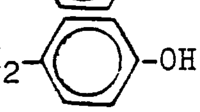 (= Tyrosin)
= $-CH_2-OH$ (= Serin)
= $-CH(OH)-CH_3$ (= Threonin)
= $-CH_2-SH$ (= Cystein)
= $-CH_2-$ 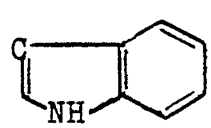 (= Tryptophan)

Die Peptidkette $R^4$ kann aus proteinogen und nicht-proteinogen Aminosäuren sowie aus einem Gemisch der beiden Gruppen zusammengesetzt sein, wobei die obengenannten Definitionen gelten.

Unabhängig davon, ob man die Racemate oder die reinen L-Verbindungen einsetzt, werden bei dem erfindungsgemäßen Verfahren allein die L-Enantiomeren der Ausgangsverbindungen zu den entsprechenden L,L-Verbindungen verknüpft.

Bei der erfindungsgemäßen Verfahrensvarianten a) geht man am besten so vor, daß man die beiden Komponenten, die Verbindungen der Formel I und II im molaren Verhältnis I:II-1:0,75 bis 1,5 in so wenig wie möglich entmineralisiertem Wasser aufnimmt und gegebenenfalls 5-60 Vol.% (bezogen auf das Volumen I

+ II + $H_2O$) eines organischen Lösungsmittels, wie Methanol, Ethanol, Dimethylsulfoxid (DMSO) oder Dimethylformamid (DMF) hinzugibt und anschließend den pH-Wert der erhaltenen Lösung auf 7 bis 9, vorzugsweise 8 einstellt. Es können sowohl die Racemate als auch die L-Verbindungen der Formel I und II eingesetzt werden. Die Verbindungen der Formel II werden vorzugsweise als reine L-Verbindungen eingesetzt. Generell lassen sich hier alle Amide von proteinogenen Aminosäuren einsetzen. Bei einer Temperatur von 25 °C bis 45 °C, vorzugsweise 28 °C bis 32 °C, wird die Umsetzung durch Hinzugabe von ca. 5-500 U/mmol, bevorzugt 10-60 U/mmol verdünnter $\alpha$- oder $\delta$-Chymotrypsinlösung gestartet. Bevorzugt wird die Reaktionslösung während der Umsetzung gerührt. Das Enzym kann entweder als solches oder z.B. fixiert an Kieselgel eingesetzt werden. Der pH-Wert der Lösung kann bevorzugt unter Verwendung eines Autotitrators, durch Hinzugabe von 0,1 N oder 1 N Natronlauge konstant gehalten werden. Die analytische Verfolgung der Reaktion kann beispielsweise mittels RP-HPLC erfolgen. Die Reaktion wird abgebrochen, wenn bei Einsatz der L-Verbindungen der Formel I kein Ester mehr nachweisbar ist. bei dem Einsatz der D,L-Verbindungen der Formel I wird die Reaktion beendet, wenn der Gehalt an Peptid (III) ein Maximum durchläuft. Das Beenden der Enzym-Reaktion kann entweder durch Zugabe von beispielsweise Ameisensäure erfolgen (wenn ein lösliches Enzym verwendet wurde) oder durch ein Abfiltrieren des fixierten (immobilisierten) Enzyms. Der so erhaltene Rohansatz wird im Eisbad abgekühlt und das ausgefallene Rohprodukt (III) abfiltriert. Die Aufarbeitung und Reindarstellung des Peptids kann beispielsweise durch Waschen der Rohsubstanz mit Wasser und/oder einem Alkohol wie Methanol, anschließender Zugabe von verdünnter Säure wie Salzsäure und schließlich Versetzen mit einer verdünnten Lauge wie Natronlauge erfolgen.

Bevorzugt werden Ansätze, die organische Lösungsmittel enthalten nach dem Stoppen der Enzymreaktion im Vakuum eingeengt - bei Verwendung von beispielsweise Methanol oder Ethanol - bzw. gefriergetrocknet - bei Verwendung von beispielsweise DMSO oder DMF. Die weitere Aufarbeitung erfolgt wie oben beschrieben.

Ansätze, deren Rohprodukte durch Auswaschen nicht zum HPLC-reinen Peptid führen, können beispielsweise durch Extraktion mit Essigsäureethylester oder Methylisobutylketon gereinigt werden.

Bei der Verfahrensvarianten b) geht man am besten so vor, daß man wäßrige Lösungen der beiden Ausgangskomponenten, der racemischen oder der L-Verbindungen der Formel IV und V, getrennt herstellt. Der pH-Wert der beiden Lösungen wird auf 5 bis 7, vorzugsweise aug 6 eingestellt. Die Molaritäten der beiden Lösungen sollten so bemessen sein, daß die Lösung der Substanz der Formel V 1,8 bis 2,2, bevorzugt 1,9 bis 2,1 mal konzentrierter ist, als die Lösung der Substanz der Formel IV. Diese beiden Lösungen werden nun synchron kontinuierlich in eine wäßrige Vorlage getropft, die 10-30 mmol/l, vorzugsweise 15-25 mmol/l eines gut löslichen Calciumsalzes wie Calciumacetat enthält und die das Enzym enthält. Als Enzym kann z.B. käufliche Thermoase eingesetzt werden. Die Enzymkonzentration beträgt bei 7,4 U/mg ca. 5-10 mg/mmol, bevorzugt 7-14 mg/mmol, bezogen auf die Konzentration an Substanz gemäß Formel V. Die Reaktionstemperatur beträgt 30 °C bis 50 °C, bevorzugt 35 °C bis 40 °C. Beim Eintropfen der Lösungen der Verbindungen der Formel IV und V in die Vorlage präzipitiert kontinuierlich das Peptid VI. Dieses wird nach vollendetem Umsatz mit verdünnter Säure, bevorzugt 2,5 N Salzsäure gewaschen, wobei das freie Peptid VI als Reinsubstanz (Festkörper) zurückbleibt.

Die erfindungsgemäß eingesetzten Enzyme $\alpha$- bzw. $\delta$-Chymotrypsin und Thermoase sind käuflich.

Die Ausgangsverbindungen der Formeln I, II, IV und V sind, sofern nicht käuflich, auf einfache Weise aus den entsprechenden Aminosäuren herstellbar.

So erhält man D,L-Aminosäureester beispielsweise durch Umsetzung der D,L-Aminosäuren mit Thionylchlorid und den entsprechenden Alkoholen. Entsprechende Verfahren sind beispielsweise beschrieben in: M. Brenner, W. Huber, Helv. Chim. Acta, 36, 1109 (1957).

Die D,L-Aminosäureamide der Formel II sind beispielsweise durch Umsetzung des Esters mit überschüssigem Ammoniak zugänglich (Houben Weyl,"Methoden der Organischen Chemie Bd. 11/2, S. 359, Georg Thieme Verlag, Stuttgart, 1958).

D,L-N-Acyl-Aminosäureester erhält man beispielsweise durch Umsetzung der D,L-Aminosäureester mit Acylanhydriden oder Acylsäurechloriden (Greenstein, Winitz, "Chemistry of the Amino Acids", Wiley, New York, 1961, oder Houben Weyl, "Methoden der Org. Chemie", Bd. 11/2, S. 30, Georg Thieme Verlag, Stuttgart, 1958 und ibid Bd. 15/1, S. 46 ff., 1974). Beispielsweise wird D,L-2-Ethoxycarbonylamino-4-phenylbuttersäureethylester (siehe Beispiel 6) aus D,L-2-Brom-4-phenyl-buttersäureethylester und KOCN in DMF/Ethanol analog dem in Chem. Ber. 114, 173 (1981) beschriebenen Verfahren dargestellt.

D,L-N-Benzoyl-(2-Furyl)-Alaninmethylester (s. Beispiel 3) wird synthetisiert, indem man beispielsweise zunächst über eine Erlenmeyer Azlactonsynthese aus Furfural, Hippursäure in Acetanhydrid/Natriumacetat das Dehydroazlacton herstellt, welches mit Natriummethanolat/Methanol ringgeöffnet wird und anschließend mit $H_2$/Tris(triphenylphosphin)-Rhodium(I)-chlorid in Methylenchlorid/Benzol zum Endprodukt hydriert wird.

Alle anderen verwendeten Ausgangsverbindungen sind entweder käuflich, oder aber es sind die jeweiligen Aminosäuren käuflich, die durch einfache Peptidverknüpfung, Veresterungs- und/oder Acylierungsreaktionen in die entsprechenden Ausgangsverbindungen überführbar sind.

Der Vorteil bei den erfindungsgemäßen Verfahrensvarianten a) und b) liegt vorallem darin, daß die Verbindungen der Formel I, II bzw. IV, V sowohl als Racemate, als auch als reine L-Verbindungen eingesetzt werden können.

In den nachfolgenden Beispielen wird die Erfindung näher erläutert und schließlich in den Ansprüchen definiert.

## Biespiele

Übersicht über die in den Beispielen benutzten Abkürzungen:

| | |
|---|---|
| Ac | Acetyl = $CH_3$-CO- |
| ac | Acetat = $CH_3COO$- |
| Ala | Alanin |
| Asp | Aspartyl (Asparaginsäure) |
| Bz | Benzoyl |
| Cbz | = Z = Benzyloxycarbonyl |
| Et | Ethyl |
| Etoc | Ethoxycarbonyl |
| Fu | Furyl |
| Gly | Glycin |
| His | Histidin |
| HPhe | Homophenylalanin |
| Leu | Leucin |
| Me | Methyl |
| Phe | Phenylalanin |
| Ph | Phenyl |
| Ser | Serin |
| Th | Thienyl |
| Tyr | Tyrosin |
| Val | Valin |

## Beispiel 1

Peptidsynthese mit α-Chymotrypsin

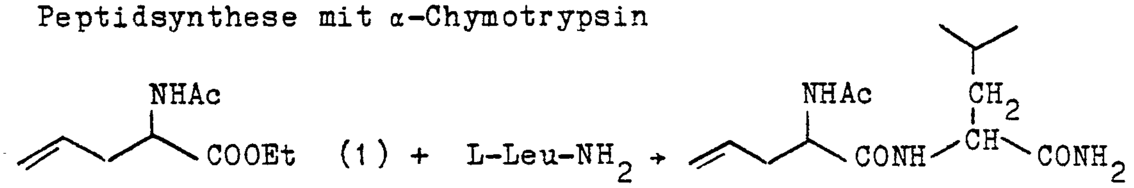


DL-N-Ac-(Allyl-Gly)-OEt
MG: [185.22]

L-N-Ac-(Allyl-Gly)-LeuNH$_2$
MG: [269.35]

Man löst 286 mg (1,5 mmol) Ester (1), 390 mg (3 mmol) Leu-$NH_2$ in 3.5 ml Wasser, 1,5 ml Ethanol, 1,1 ml 1N NaOH, korrigiert den pH auf 8.00 und erwärmt auf 30 °C. Es werden ca. 1.5 mg α-Chymotrypsin hinzugegeben und während der Peptidsynthese mit 1N NaOH-Lösung titriert. Nach ca. 5h erreicht man ein Produktmaximum. Der Niederschlag wird in Wasser aufgenommen und mit dem gleichen Volumen Essigsäureethylester extrahiert. In der Essigsäureethylester-Phase reichert sich das Peptid stark an. Wiederholung der Extraktion bis die EE-Phase nur noch Peptid enthält.
Zusammensetzung der Rohlösung:
Peptid:D-Ester:Säure = 66:27:7
Ausbeute Peptid: 102 mg (51 %)
Charakterisierung:
$^1$H-400 MHz-NMR ($d_6$-DMSO);
0,81 - 0,90 m, 6 H,

, 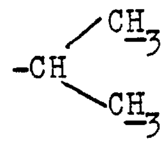

1,48 m, 2H, $CH_2$-CH<
1,55 m, 1H, C-CH<
1,84 s, 3H, $COCH_3$
2,22 m, 1H, 2,48 m, 1H, C=C-$CH_2$-
4,20 m und 4,28 m, 2H, 2 x NH-CH-
5,08 m, 2H, $_2$HC=CH-
5,75 m, 1H, $_2$HC=CH-
6,94 s, 1H, 7,18 s, 1H, -$NH_2$

7,80 d, J = 8 Hz, 1H
7,97 d, J = 8 Hz, 1H  CO-$NH_2$

**Beispiel 2**

**Peptidsynthese mit α-Chymotrypsin, immobilisiert an Kieselgel:**

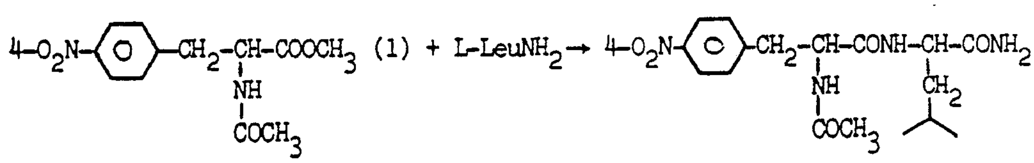

DL-Ac-(4-$NO_2$-Phe)-OMe
MG: [269]

L-Ac-(4-$NO_2$-Phe)-Leu-$NH_2$
MG: [364.40]

Man löst 1,13 g (4,2 mmol) Ester (1), 0,52 g (4 mmol) Leu-$NH_2$ in 2 ml Wasser, 4 ml $CH_3OH$, 2,5 ml 1N NaOH und 3,5 ml DMSO. Wird die homogene Lösung mit α-Chymotrypsin oder δ-Chymotrypsin inkubiert, so läuft die Peptidsynthese nur wenige Minuten und bricht mit unvollständigem Umsatz ab. Dieses Phänomen tritt nicht auf bei Verwendung von ca. 1 ml α-Chymotrypsin-Keiselgel.
Nach 4 h erzielt man maximale Peptidkonzentration (Nachweis in HPLC). Die Abtrennung des Katalysators von ausgefallenen Produkt erfolgt durch Sedimentation. Das Reaktionsprodukt wird mehrmals in Wasser aufgenommen und jeweils gefriergetrocknet. Der feste Rückstand (Zusammensetzung: Peptid:D-Ester:Säure = 58:29:13) wird mit Wasser/Methanol vorsichtig ausgewaschen, bis HPLC-reines Peptid als Rückstand verbleibt.
Reines Peptid: 310 mg (41 % d.Th.)
Charakterisierung von L-Ac-(4-$NO_2$-Phe)-Leu-$NH_2$:
$^1$H-400 MHz-NMR:
0,79 - 0,84 m, 6H,

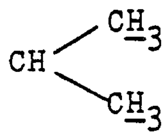

1,49 m, 2H, $>CH-CH_2-CH<$
1,60 m, 1H,

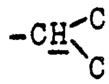

1,75 s, 3H, $COCH_3$
2,84 m, 1H, 3,13 m, 1H

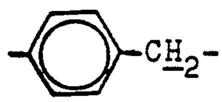

4,20 m, 1H, -CH-NH-
4,58 m, 1H, -CH-NH-
6,94 s, 1H, 7,23 s, 1H $-NH_2$
7,48 m, 2H, 2H-Aryl
8,02-8,18 m, 4H, 2H-Aryl + 2 x NH-CO

**Beispiel 3**

Peptidsynthese mit δ-Chymotrypsin:
L-N-Bz-(2-Fu-Ala)-$LeuNH_2$

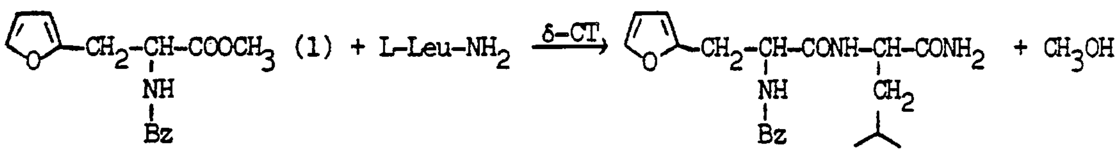


DL-N-Bz-(2-Fu-Ala)-$OCH_3$

L-N-Bz-(2-Fu-Ala)-L-$LeuNH_2$
MG: [371.44]

Man löst 109 mg (0.4 mmol) Ester (1) und 100 mg L-Leu-$NH_2$ (0,76 mmol) in 1 ml Methanol, 1 ml Wasser und 1,5 ml DMSO bei pH = 8,00 (30 °C). Zur homogenen Lösung gibt man 8 mg δ-Chymotrypsin und hält den pH mit 1 N NaOH auf 8,00. Nach ca. 10 Min. Reaktionszeit beginnt das Produkt zu präzipitieren. Nach 30 Min. erreicht die Produktkonzentration im HPLC ein Maximum, worauf die Reaktion mit wenigen Tropfen Ameisensäure abgebrochen wird. Nach Abkühlen des Ansatzes wird abgefrittet und 2 mal mit Wasser nachgewaschen.
Ausbeute: 92 mg Substanz.

| Zusammensetzung: | | |
|---|---|---|
| Substanz | Festkörper % | Filtrat % |
| Peptid | 83 | 1 |
| N-Bz-(2-Fu-Ala)-OMe | 12 | 34 |
| N-Bz-(2-Fu-Ala)-OH | 5 | 4 |
| Leu-$NH_2$ | - | 61 |

Durch vorsichtiges Umkristallisieren des Festkörpers in Methanol:$H_2O$ = 1:1 gewinnt man 49,2 mg (66 %) reines Peptid.

Beim langsamen Verdunsten des Filtrats fällt kontinuierlich D-N-Bz-(2-Fu-Ala)-$OCH_3$ als Festkörper an: 37 mg (68 % d.Th.) rückgewonnen.

Charakterisierung von L-N-Bz-(2-Fu-Ala)-L-Leu$NH_2$:

$^1$H-400 MHz-NMR in ($d_6$-DMSO):

0,81-0,87 m, 6H, 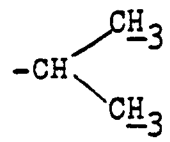

1,47 m, 2H, >CH-C$\underline{H}_2$-CH<

1,62 m, 1H, -C$\underline{H}$<$^C_C$

3,18 m, 2H, 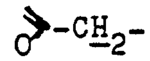

4,25 m, 1H, NH-C$\underline{H}$-CO

4,75 m, 1H, $CH_2$-C$\underline{H}$-CO

6,17 m, 1H, 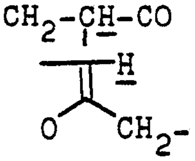

6,32 m, 1H, 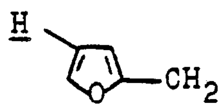

6,97 s, 1H, 7,23 s, 1H, N$\underline{H}_2$

7,43-7,55, m, 4H, 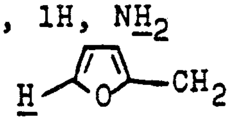 + 3H-Benzol (m,m',p)

7,79-7,82, m, 2H, o,o'-$\underline{H}$-Benzol

7,93 d, J = 8 Hz, 1H, N$\underline{H}$-CO

8,57 d, J = 8 Hz, 1H, N$\underline{H}$-CO

**Beispiel 4**

Kurzform

L-Z-Asp + 2 DL-(4-F-Phe)-OMe —Thermoase, Ca(ac)$_2$→ [L-Z-Asp-L-(4F-Phe)-$OCH_3$ x D-4F-Phe-$OCH_3$] + $H_2O$

MG: [267] MG: [197] MG: [643]

[L-Z-Asp-L-(4F-Phe)-$OCH_3$ x D-4F-Phe-$OCH_3$] + HCl → L-Z-Asp-L-(4F-Phe)-$OCH_3$ + D-4F-Phe-OMe x HCl

MG: [446]

**Ansatz:**

6,8 g (25 mmol) L-Z-Asp
10,0 g (50 mmol) DL-4-F-Phe-OMe
40 ml Wasser
200 mg Thermoase, 100 mg Calciumacetat

**Verfahren:**

Man löst die beiden Komponenten in Wasser, stellt den pH mit konzentrierter NaOH auf 6.00 ein und gibt dann Enzym und Ca(ac)$_2$ zu. Man rührt 16 h bei 22° C.
Der ausgefallene Niederschlag wird abgesaugt, mit 20 ml 2,5 N HCl suspendiert (Umsalzprozeß), wiederum abgesaugt, mit Wasser nachgewaschen und getrocknet.
Auswage: 8,26 g L-Z-Asp-L-4-F-Phe-$OCH_3$ (74 % d.Th.).
Charakterisierung:
$^1$H-400 MHz-NMR $d_6$-DMSO:
2,37-2,62 m, 2H, -$C\underline{H}_2$-COOH
2,88 - 3,33 m, 2H,

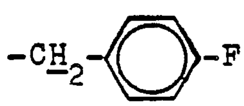

3,58 s, 3H, $COOCH_3$
4,33 - 4,47 m, 2 x - CH-
5,01 s, 2H,

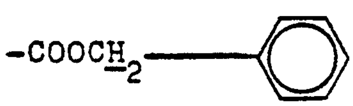

7,03 - 7,52 m, 11H, 10 H-Aryl + NH
8,25 d, J = 8 Hz, 1H, NH
12,31 s, 1H, COOH

**Beispiel 5**

L-Z-Asp + 2 DL-(2-Th-Ala)-OMe x HCl →
MG: [267] MG: [221.7]

[L-Z-Asp-L-(2-Th-Ala)-OMe x D-(2-Th-Ala)-OMe] + $H_2O$
MG: [692]

Umsalzen

[L-Z-Asp-L-(2-Th-Ala)-OMe x D-(2-Th-Ala)-OMe] + $H_2O$ HCl

L-Z-Asp-L-(2-Th-Ala)-OMe + D-(2-Th-Ala)-OMe
MG: [470.7]

Ansatz:

5.34 g (20 mmol) L-Z-Asp
8,90 g (40 mmol) DL-(2-Th-Ala)-OMe x HCl
180 mg Thermoase,
110 mg $Ca(ac)_2$

Verfahren:

L-Z-Asp wird in 10 ml Wasser aufgenommen und mit 10 N NaOH auf pH 6,00 eingestellt (≙ Lsg I). Analog wird eine Lösung für den Ester zubereitet (≙ Lsg II). Dann werden Thermoase und $Ca(ac)_2$ in 2 ml $H_2O$ gelöst und bei 40 °C vorgelegt. Anschließend tropft man innerhalb von 2 h die Lösungen I + II synchron in die Vorlage. Der pH wird im Autotitrator mit 1 N NaOH auf pH 6.00 gehalten.

Nach 7 h bei 40° filtriert man den Niederschlag ab und läßt das Filtrat noch 16 h nachreagieren. Die wiederum ausgefallene Substanz wird mit dem ersten Niederschlag vereinigt, mit 20 ml 2,5 N HCl suspendiert, abfiltriert, mit Wasser gewaschen und getrocknet.

Auswaage: 7,7 g (82 % d.Th.) Peptid
Charakterisierung von L-Z-Asp-L-(2-Th-Ala)-OMe
$^1H$-400 MHz-NMR ($d_6$-DMSO):
2,42-2,68 m, 2H, $-CH_2-COOH$
3,14 - 3,35 m, 2H,

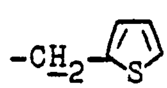

3,62 s, 3H, $COOCH_3$
4,37 - 4,50 m, 2H, 2 x -CH-

5,03 s, 2H, $-COOCH_2-Ph$
6,90 - 6,95 m, 2H, 2H Thiophen
7,31-7,40 m, 6H, 1H Thiophen + 5H Phenyl
7,60 d, J = 8,2 Hz, 1H, -NH-
8,31 d, J = 7,5 Hz, 1H, -NH-
12,29 s, 1H, -COOH

In einer analogen Verfahrensweise zu Beispiel 5 läßt sich beim Einsatz von D,L-(2-Fu)-Ala-$OCH_3$ das Peptid: L-Z-Asp-L-(2-Fu)-Ala-$OCH_3$ darstellen.
Ausbeute: 40 % d.Th.
Charakterisierung von L-Z-Asp-L-(2-Fu)-Ala-$OCH_3$:
$^1$H-NMR (400 MHz $d_6$-DMSO):
2,38-2,70; m, 2H, $-CH_2-COOH$
3,03-3,08; m 2H,

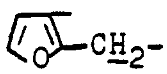

3,60; S, 3H, $COOCH_3$
4,32-4,55; m, 2H, 2 x - CH-
5,05; s, 2H, $COOCH_2-Ph$
6,15; m, 1H; 6,35; m, 1H, 2H-Furan
7,35; m, 6H, 1H-Furan und 5H-Phenyl
7,50; s, COOH (teilweise verdeckt)
7,55; d, J = 8Hz, 1H, -NH-CO-
8,30; d, J = 8 Hz, 1H, -NH-CO-

**Beispiele 6 bis 13**

Peptidsynthesen mit Chymotrypsin bei pH = 8,00 unter Verwendung der Acylkomponente

$$R^1-\underset{\displaystyle NH-R^2}{CH}-COOR^3$$

und L-Leu-$NH_2$ als Aminacceptor.

In der nachfolgenden Tabelle 1 sind die Ausgangsacylkomponente (I) mit den Substituenten $R^1$, $R^2$ und $R^3$, die Konzentration von (I), die Konzentration von L-Leu-$NH_2$, das verwendete Lösungsmittelgemisch, die Enzymmenge, die Reaktionstemperatur, Reaktionsdauer, prozentuale Ausbeute sowie das Analogbeispiel angegeben. Die Tabelle 2 enthält $^1$H-NMR-spektroskopische Daten der Substanzen aus den Beispielen 6 bis 13.

Erläuterungen zu Tabelle 1:

n-Prop. = n-Propyl
α-CT-Ⓚ = α-Chymotrypsin fixiert an Kieselgel
δ-CT = δ-Chymotrypsin

* = Reaktion bei pH = 8,70
** = Reinigung des Peptids durch Sublimation bei $10^{-3}$ Torr

**Tabelle 1**

$R^1$-CH-$COOR^3$
NH-$R^2$ (I)

| Bsp. | $R^1$ | $R^2$ | $R^3$ | I g/mmol | Leu-$NH_2$ g/mmol[2] | Lösungsmittel ml | mg/Enzym | Temp. °C | Reakt. dauer (h) | Ausb. Peptid % | Durchführung Aufarbeitung analog Bsp.: |
|---|---|---|---|---|---|---|---|---|---|---|---|
| 6 * | 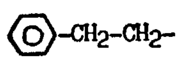 | $-COOC_2H_5$ | Et | 6,69 (25) | 3,25 (25) | 10 ml Ethanol<br>35 ml $H_2O$<br>15 ml DMSO | 4/δ-CT | 30 | 12 | 51 | 3 ** |
| 7 | 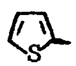 | Ac | n-Prop. | 0,127/0,53 | 0,195/1,5 | 0,25 n-Propanol<br>0,5 DMSO<br>0,5 $H_2O$ | 1,5/δ-CT | 30 | 4 | 64 | 3 |
| 8 | 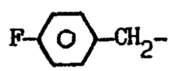 | Ac | Me | 0,119/0,50 | 0,098/0,75 | 1,2 Methanol<br>2,4 $H_2O$ | 1/δ-CT | 30 | 5 min | 62 | 3 |
| 9 | 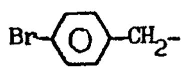 | Ac | n-Prop. | 0,150/0,5 | 0,098/0,75 | 1,5 ml Methanol<br>1,5 ml $H_2O$<br>0,25 DMSO | 1/δ-CT | 30 | 10 min | 61 | 3 |
| 10 | 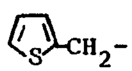 | Ac | Me- | 0,45/2 | 0,26/2 | 2 ml Methanol<br>2 ml $H_2O$<br>1,25 1N NaOH | 2/δ-CT | 30 | 1,5 | 28 | 3 |
| 11 | 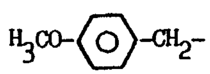 | Ac | Me- | 0,500/2 | 0,26/2. | 3 ml Methanol<br>4 ml $H_2O$<br>0,5 ml DMSO | 2/δ-CT | 30 | 4 | 51 | 3 |
| 12 | 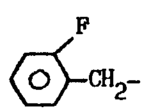 | Cbz- | Me- | 1,00/3,00 | 0,59/4,50 | 2 ml Methanol<br>3 ml DMSO<br>2 ml $H_2O$ | 5 ml α-CT-(K) | 35 | 32 | 31 | 2 |
| 13 | 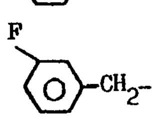 | Cbz- | Me- | 1,00/3,00 | 0,59/4,50 | 3 ml Methanol<br>3 ml DMSO<br>3 ml $H_2O$ | 5 ml α-CT-(K) | 35 | 32 | 18 | 2 |

**Tabelle 2**

$R^1-CH-CONH-CH-CONH_2$
$NH-R^2 \quad R^4$ (II)

| Bsp. | $R^1$ | $-\underline{CH}-CONH-\underline{CH}-$ | $R^2$ | $-CONH-$ $-\underline{NH}-R_2-$ | $CON\underline{H_2}$ | $R^4$ |
|---|---|---|---|---|---|---|
| 6 | 1.87 m, 2H ) $-\underline{CH_2}-\underline{CH_2}$<br>2.60 m, 2H )<br>7.14-7.33, m,<br>H-Aryl<br>verdeckt | ~ 4.00, m, verdeckt<br>4,24 m, 1H | 1.15 t, J = 7 Hz,<br>$-OCH_2\underline{CH_3}$<br>3.98 q, J = 7 Hz,<br>$-O\underline{CH_2}-CH_3$<br>verdeckt | 7.80, d, J = 8 Hz<br>1H<br>Restsignale verdeckt | verdeckte<br>Signale<br>im Aromaten-<br>bereich | 0.86-0.89 m, 6H<br>1,44 m, 2H<br>1,61 m, 1H |
| 7 | 6,95 m, 1H)<br>7,23 m, 1H) 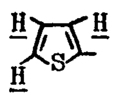<br>7,44 m, 1H) | 5,70, d, J = 8 Hz,<br>1H<br>4,24, m, 1H | 1,89, s, 3H | 8,35, d, J = 8 Hz<br>8,56, d, J = 8 Hz | 7,24, s̄,<br>2H | 0,83-0,87 m, 6H<br>1,45, m, 2H<br>1,61, m, 1H |
| 8 | 7,05, 2H, ) AA'BB'<br>7,23, 2H, )<br><br>2,70 m, 1H )<br>2,96 m, 1H ) 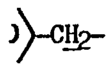 | 4,20 m, 1H<br>4,48 m, 1H | 1,73, s, 3H | 7,91, d, J = 8 Hz<br>8,04, d, J = 8 Hz | 6,95, s̄, 1H<br>7,17, s̄, 1H | 0,84-0,87 m, 6H<br>1,44, m, 2H<br>1,57, m, 1H |
| 9 | 7,19, 2H ) AA'BB'<br>7,43, 2H )<br><br>2,70 m, 1H )<br>2,95 m, 1H ) 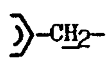 | 4,50, m, 1H<br>4,21, m, 1H | 1,73, s, 3H | 7,92, d, J = 8 Hz<br>8,17, d, J = 8 Hz | 6,93, s̄, 1H<br>7,20, s̄, 1H | 0,83-0,87, m, 6H<br>1,44, m, 2H<br>1,60, m, 1H |
| 10 | 6,90 m, 2H) 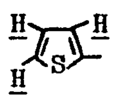<br>7,28 m, 1H)<br><br>3,03 m, 1H)<br>3,20 m, 1H) 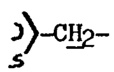 | 4,23, m, 1H<br>4,48, m, 1H | 1,78, s, 3H | 7,91, d, J = 8 Hz<br>8,12, d, J = 8 Hz | 6,90, s̄, 1H,<br>7,16, s̄, 1H | 0,82-0,89, m, 6H<br>1,42, m, 2H<br>1,61, m, 1H |

Tabelle 2 (Fortsetzung)

| Bsp. | $R^1$ | -CH-CONH-CH- | $R^2$ | -CONH- -NH-R2- | $CONH_2$ | $R^4$ |
|---|---|---|---|---|---|---|
| 11 | 6,80, 2H, ) AA'BB'<br>7,14, 2H, )<br>2,65 m, 1H )<br>2,91 m, 1H ) 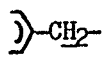 | 4,18, m, 1H<br>4,42, m, 1H | 1,77, s, 3H | 7,83, d, J = 8 Hz 2H<br>7,99, d, J = 8 Hz 2H | 6,90, s, 1H<br>7,18, s, 1H | 0,84-0,87 m, 6H<br>1,41, m, 2H<br>1,60, m, 1H |
| 12 | 7,08-7,40, $H_{ARYL}$<br>2,80, m; 1H )<br>3,08, m, 1H ) 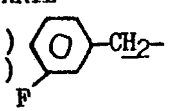 | 4,20- ) teilweise<br>4,43 m ) verdeckt | 7,08-<br>7,40 $H_{ARYL}$<br>4,94, s, 2H | 7,58, d, J = 8 Hz 1H<br>7,96, d, J = 8 Hz 1H | verdeckte Signale | 0,85-0,88 m, 6H<br>1,43, m, 2H<br>1,62, m, 1H |
| 13 | 7,00-7,38, $H_{ARYL}$<br>3,01, m, 1H)<br>3,05, m, 1H) 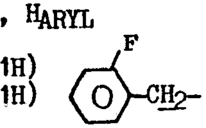 | 4,18- m, 1H<br>4,42 m, 1H | 7,00-<br>7,38 $H_{ARYL}$<br>5,00, s, 2H | 7,60, d, J = 8 Hz 1H<br>7,96, d, J = 8 Hz 1H | Signale unter $H_{ARYL}$ | 0,80-0,88 m, 6H<br>1,39, m, 2H<br>1,66, m, 1H |

## Ansprüche

1. Verfahren zur enzymatischen Verknüpfung von Aminosäuren und/oder Aminosäurederivaten, wovon mindestens eine nichtproteinogen ist, dadurch gekennzeichnet, daß man

a) eine D,L- oder L-Verbindung der Formel I,

$$HB{-}R^2N{-}\underset{R^1}{\underset{|}{HC}}{-}COOR^3 \qquad (I)$$

in der
$R^1$ ein Rest der Formel

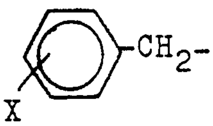

bedeutet, in der
X ein Halogen in ortho- und/oder meta- und/oder para-Position oder ein Nitro- oder Methoxy-Rest in para-Position ist,
oder einen Rest der Formel

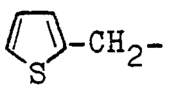, 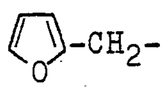, 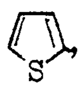, 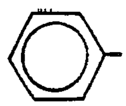, 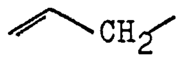 oder

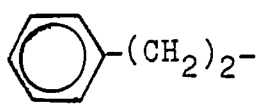 darstellt,

$R^2$ Wasserstoff oder ein Rest der Formel A-CO- ist, worin A Benzyloxy, $C_1$-$C_4$-Alkyl, Phenyl oder $C_1$-$C_4$-Alkoxy bedeutet,
$R^3$ $C_1$-$C_4$-Alkyl oder Benzyl

$$B\ (HN{-}\underset{R^4}{\underset{|}{CH}}{-}CO)_n \text{ bedeutet,}$$

wobei n 0-10 und $R^4$ eine Peptidkette ist,
mit einer D,L- oder L-Verbindung der Formel II,

$$H_2N - \underset{R^5}{\underset{|}{CH}} - CO - BR^6 \qquad (II)$$

in der
$R^5$ Wasserstoff, $C_1$-$C_4$-Alkyl oder einen anderen Rest einer proteinogenen Aminosäure bedeutet,
$R^6$ eine Aminogruppe, -NH-($C_1$-$C_4$)-Alkyl oder eine Phenylaminogruppe ist und
B die obengenannte Bedeutung hat in Gegenwart von Chymotrypsin zu einer L,L-Verbindung der Formel III,

$$HB - R^2N - \underset{R^1}{HC} - CONH - \underset{R^5}{CH} - CO - BR^6 \qquad (III)$$

in der $R^1$, $R^2$, $R^5$, $R^6$ und B die obengenannte Bedeutung haben, umsetzt oder

b) eine D,L- oder L-Verbindung der Formel IV,

$$HB - R^8N - \underset{R^7}{CH} - COOH \qquad (IV)$$

in der
$R^7$ ein Rest der Formel $HOOC-(CH_2)m-$ bedeutet, worin m 1 oder 2 ist,
$R^8$ Wasserstoff, $C_1$-$C_4$-Alkyloxycarbonyl oder Benzyloxycarbonyl ist und
B die obengenannte Bedeutung hat
mit einer D,L- oder L-Verbindung der Formel V,

$$H_2N - \underset{R^{10}}{CH} - CO - B - R^9 \qquad (V)$$

in der
$R^{10}$ einen Rest der Formel

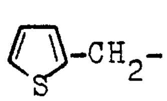 , 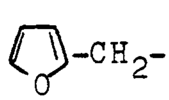 oder 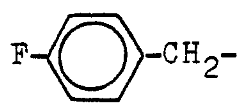

darstellt,
$R^9$ $C_1$-$C_8$-Alkoxy, $NH_2$, NH-($C_1$-$C_4$)-Alkyl oder eine Phenylaminogruppe bedeutet,
in Gegenwart von Thermolysin zu einer L,L-Verbindung der Formel VI,

$$HB - R^8 - N - \underset{R^7}{CH} - CONH - \underset{R^{10}}{CH} - B - R^9 \qquad (VI)$$

in der $R^7$, $R^8$, $R^9$, $R^{10}$ und B die oben angegebene Bedeutung haben, umsetzt.

2. Verfahren nach Anspruch 1, dadurch gekennzeichnet, daß mindestens eine der nachfolgenden Bedingungen erfüllt ist:
$R^2$ bedeutet Wasserstoff, Benzoyl, Benzyloxycarbonyl, Acetyl oder Ethoxycarbonyl
$R^3$ bedeutet Ethyl, Methyl oder n-Propyl, die Verbindung der Formel II ist L-Leu-$NH_2$,
$R^7$ bedeutet $HOOC-CH_2-$ ,
$R^8$ bedeutet Wasserstoff oder Benzyloxycarbonyl,
$R^9$ bedeutet Methoxy.

3. Verfahren nach Anspruch 1 oder 2, dadurch gekennzeichnet, daß bei der Verfahrensvarianten a) der pH-Wert auf 7 - 9 eingestellt wird und bei der Verfahrensvarianten b) der pH-Wert auf 5 - 7 eingestellt wird.

4. Verfahren nach einem oder mehreren der Ansprüche 1 bis 3, dadurch gekennzeichnet, daß die Verfahrensvariante a) bei einer Reaktionstemperatur von 25° C bis 45° C und die Verfahrensvariante b) bei einer Reaktionstemperatur von 30° C bis 50° C durchgeführt wird.

5. Verfahren nach einem oder mehreren der Ansprüche 1 bis 4, dadurch gekennzeichnet, daß bei der Verfahrensvariante a) die L-Verbindungen der Formel II und bei der Verfahrensvariante b) die L-Verbindungen der Formel IV eingesetzt werden.

6. Verwendung der gemäß einem oder mehreren der Verfahren nach den Ansprüchen 1 bis 5 erhältlichen Peptide zur Herstellung von Enkephalinen, Bradykinen, Renin- oder ACE-Inhibitoren.

7. Verbindungen der Formel

L-Z-Asp-L-(2-Th-Ala)-$OCH_3$

L-N-Bz-(2-Fu-Ala)-L-Leu-$NH_2$ und

L-Ac-(2-Th-Ala)-L-Leu-$NH_2$.